# EUROPÄISCHE PATENTSCHRIFT

(11) **EP 1 631 206 B1**
(45) Veröffentlichungstag und Bekanntmachung des Hinweises auf die Patenterteilung: **10.10.2007**
(21) Anmeldenummer: 03817232.6
(22) Anmeldetag: 12.06.2003
(51) Int. Cl.: A61B 17/72

(54) **CHIRURGISCHER NAGEL**
SURGICAL NAIL
CLOU CHIRURGICAL

(43) Veröffentlichungstag der Anmeldung: 08.03.2006
(73) Patentinhaber: Synthes GmbH, 4436 Oberdorf (CH)
(72) Erfinder: SCHLIENGER, André, CH-4142 Münchenstein (CH); BUETTLER, Markus, CH-4702 Oensingen (CH); SENN, Peter, CH-4437 Waldenburg (CH)
(74) Vertreter: Lusuardi, Werther
(86) Internationale Anmeldenummer: PCT/CH2003/000376
(87) Internationale Veröffentlichungsnummer: WO 2004/110291

(56) Entgegenhaltungen:
- WO-A-93/15679
- WO-A-98/46169
- US-A- 5 935 127
- US-A1- 2002 173 792

## Beschreibung

Die Erfindung betrifft einen chirurgischen Nagel, insbesondere einen intramedullären Marknagel, gemäss dem Oberbegriff des Patentanspruchs 1.

Die Verriegelung von Marknägeln gehört zum Stand der Technik. Die Einführung der Verriegelungsschrauben, oder Verriegelungsbolzen (im folgenden wird nur noch der Begriff Verriegelungsschraube verwendet, der aber auch den Begriff Verriegelungsbolzen mitumfassen soll) in die Querbohrungen eines Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - ein gewisses Spiel auf relativ zur Querbohrung.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmente bewegen können. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube verschieben kann.

Aus der US 6,296,645 HOVER ET AL. ist ein hohler, intramedullärer Marknagel aus Metall bekannt, der in den als Fenster bezeichneten sich diametral gegenüberstehenden Mantelöffnungen der Querbohrung einen oder zwei Kunststoffeinsätze aufweist, durch welche die Verriegelungsschraube eingeführt werden kann. Nachteilig bei diesem bekannten Marknagel ist der Umstand, dass die fensterartigen Kunststoffeinsätze leicht eingedrückt werden können, so dass die erwünschte Funktion verloren geht. Aber selbst bei einer sehr vorsichtigen Manipulation dürften die beiden Kunststoffeinsätze beim Durchführen der Verriegelungsschraube aus ihrem "Fenster" gedrückt werden, was ebenfalls zu einem Verlust der Funktion führt.

Weitere Marknägel mit Einsätzen sind aus der WO-A-98/46169 und der US-A-2002/0173792 bekannt.

Hier will die Erfindung Abhilfe schaffen. Der Erfindung liegt die Aufgabe zugrunde, einen chirurgischen Nagel, insbesondere einen intramedullären Marknagel zu schaffen, mit dem das vorhandene Spiel zwischen ihm und der Verriegelungsschraube risikolos eliminiert werden kann und eine verbesserte Haltekraft sowie ein verbesserter Führungseffekt zwischen Verriegelungsschraube und Marknagel erzielt werden kann.

Die Erfindung löst die gestellte Aufgabe mit einem chirurgischen Nagel, welcher die Merkmale des Anspruchs 1 aufweist.

Damit sind folgenden Vorteile erzielbar:
a) die Zielgenauigkeit bei der Einbringung der Verriegelungsschraube ist unbeeinträchtigt;
b) der Arzt kann noch intraoperativ wählen, ob er eine winkelstabile Verriegelung der Verriegelungsschraube einsetzen will oder nicht, wobei der Begriff "winkelstabil" eine Einschränkung gewisser Freiheitsgrade bedeutet;
c) Möglichkeit der winkelstabilen Fixierung des Knochenfragments in gewissen Richtungen für einen bestimmten Betrag der Last; und
d) Nagel und Einsatz können separat steril verpackt werden und der Chirurg kann wählen ob er den Nagel ohne Einsatz öder mit Einsatz verwenden will. In letzterem Fall kann der Chirurg den Einsatz selbst in Nagel einführen und gegebenenfalls auch wieder entfernen. Verwendet der Chirurg den Nagel ohne Einsatz so bleibt dieser weiterhin steril verpackt für eine nächste Verwendung.

Bei einer besonderen Ausführungsform gehorcht die Länge L des Einsatzes der Bedingung L > 0,5 D und vorzugsweise L = D.

Bei einer weiteren Ausführungsform ist der Einsatz im wesentlichen kongruent zur Querbohrung.

Bei einer besonderen Ausführungsform kann der Einsatz eine zu seiner Längsachse koaxiale Bohrung aufweist, um das Einführen der Verriegelungsschraube zu erleichtern.

Vorteilhafterweise weist Material m des Einsatzes eine Zugfestigkeit f_{z} < F_{z}, eine Druckfestigkeit f_{d} < F_{d} sowie ein Elastizitätsmodul e < 0,8 E, vorzugsweise e < 0,7 E auf.

Bei einer Ausführungsform ist das Material m des Einsatzes ein biokompatibler Kunststoff, vorzugsweise ein Polyethylen oder ein hochmolekulares Polyethylen (HMWPE), was den Vorteil mit sich bringt, dass kein Abbau des Kunststoffs mit unbekannten Abbauprodukten erfolgt.

Eine bevorzugte Weiterbildung besteht darin, dass der Einsatz aus einem Material geringerer Härte ein bioresorbierbares Polymer, vorzugsweise ein Polylactid ist. Bei dieser Ausführung resultiert anfänglich eine spielfreie Querverriegelung des Marknagels, welche dann mit zunehmender Resorption des Polymers sukzessive wieder aufgehoben wird, so dass die Querverriegelungsschraube relativ zum Marknagel und somit auch die versorgten Knochenfragmente wieder beweglich werden. Es erfolgt somit eine Dynamisierung der Knochenfragmente nach erfolgter Frakturkonsolidierung. Das bioresorbierbare Material hat den Vorteil, dass die beim Hindurchschrauben einer Verriegelungsschraube durch den Nagel entstehenden Späne vom Körper abgebaut werden können. Ein weiterer Vorteil besteht in der Möglichkeit eine eventuell zeitlich unterschiedliche Stärke der winkelstabilen Verriegelung der Verriegelungsschraube zu realisieren, d.h. einen graduellen Abbau der Haltekraft zu erreichen.

Die Querbohrung des Marknagels kann entweder eine Kreisbohrung sein, wobei das Querschnittsprofil F die maximalen Längen a = b aufweist (d.h. a = b ist der Durchmesser Querbohrung) oder auch als Langloch ausgebildet sein, wobei dann das Querschnittsprofil F die maximalen Längen a > b aufweist.

Das Material m des Einsatzes weist vorteilhafterweise auch eine geringere Dichte ρ1 als das Material M mit der Dichte ρ2 auf, wobei vorzugsweise ρ1 < 0,8 ρ2 ist.

Die durch den Einsatz hindurchführbare Verriegelungsschraube, bzw. Verriegelungsbolzen sollte vorzugsweise einen Schaft mit dem Durchmesser d aufweisen (maximaler Durchmesser, der auch ein allfälliges Aussengewinde mitumfasst), welcher den Bedingungen a > d < b gehorcht. Der Durchmesser d des Schraubengewindes sollte dabei vorzugsweise mindestens 5 % kleiner sein als die kleinere der beiden Dimensionen a, b.

Bei einer speziellen Ausführungsform erweitert sich die Querbohrung gegen die Oberfläche des Nagels hin, vorzugsweise in Form eines konischen Abschnittes. Dies hat den Vorteil, das ein darin eingeführter Einsatz mit einem dazu korrespondierenden konischen Abschnitt sich nicht mehr axial in Einführrichtung verschieben kann.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht eines durchgängig kannulierten Marknagels mit einer Querbohrung und einen dazu passenden Einsatz sowie eine Querverriegelungsschraube;
Fig. 2 einen Längsschnitt durch den Marknagel nach Fig. 1;
Fig. 3 einen um 90° verdrehten Längsschnitt durch den Marknagel nach Fig. 1;
Fig. 4 eine vergrösserte schematische Ansicht des Profils der Querbohrung des Marknagels nach Fig. 1;
Fig. 5 einen einteiligen Einsatz mit einer Längsbohrung für die Querbohrung im Marknagel, welcher nach dem Einsetzen in etwa mit der Nagelkannulierung fluchtet;
Fig. 6 einen partiellen Längsschnitt durch einen weiteren, modifizierten Marknagel mit einem modifizierten einteiligen Einsatz; und
Fig. 7 einen partiellen Längsschnitt durch einen weiteren, modifizierten Marknagel mit einem modifizierten einteiligen Einsatz.

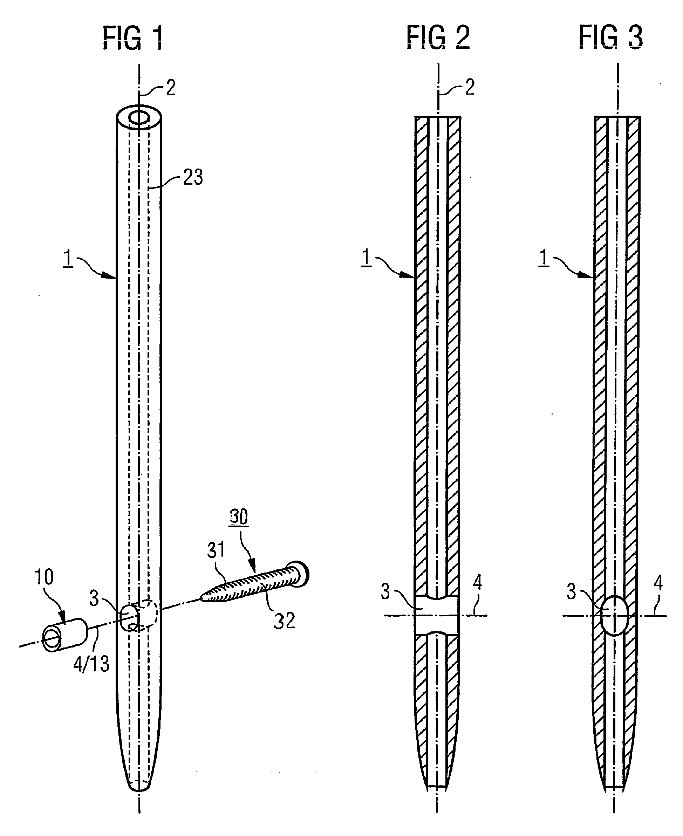

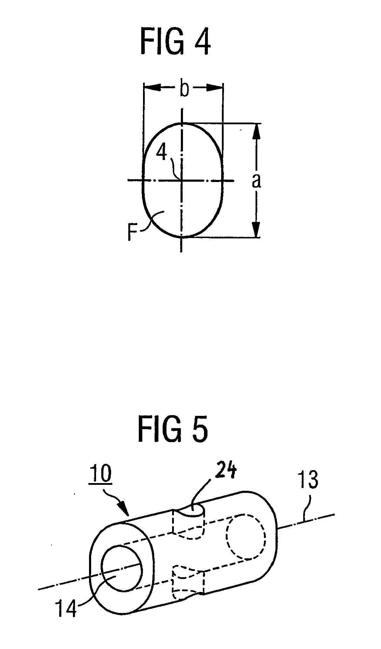

Der in den Fig. 1 - 3 dargestellte chirurgische Nagel 1 ist ein intramedullärer Marknagel für Röhrenknochen mit einer Zentralachse 2, der aus einem Metall oder einer Metalllegierung, d.h. einem Material mit relativ hoher Festigkeit (Zugfestigkeit F_{z}, Druckfestigkeit F_{d} und Elastizitätsmodul E) besteht. Der Nagel 1 besitzt eine zur Zentralachse 2 verlaufende, als Langloch mit dem Querschnittsprofil F ausgebildete Querbohrung 3 mit der Querachse 4. Wie in Fig. 4 dargestellt weist die Querbohrung 3 ein Querschnittsprofil F auf, welches in Richtung der Zentralachse 2 eine maximale Länge a und senkrecht dazu eine maximale Breite b aufweist. Der Nagel kann auch noch weitere - zeichnerisch nicht dargestellte Querbohrungen (rund oder oval) aufweisen.

Wie in Fig. 1 dargestellt, ist ein Einsatz 10 zur Einführung in die Querbohrung 3 vorgesehen. Die Dimensionierung des Einsatzes 10 ist kongruent zur Querbohrung 3, bzw. ist derart gewählt dass bei der Insertion ein Presssitz resultiert, wodurch ein Herausfallen des Einsatzes aus der Querbohrung 3 verhindert wird. Der Einsatz 10 besteht aus Material m geringerer Festigkeit, insbesondere einem geringeren E-Modul (im Vergleich zum Material M des Marknagels).

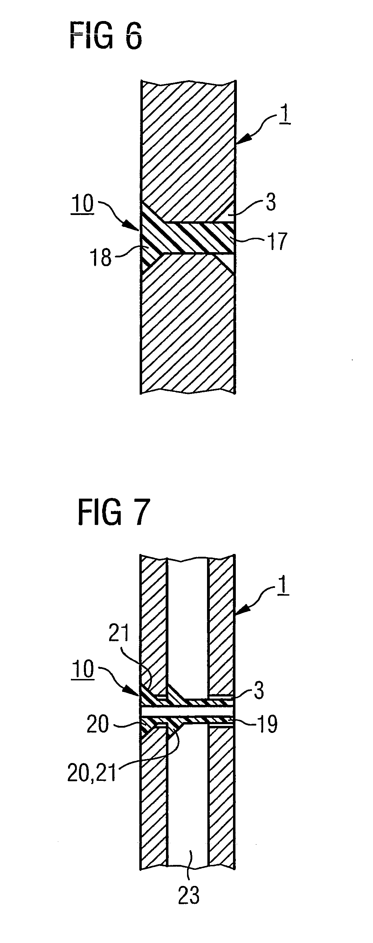

Wie in Fig. 5 dargestellt kann der Einsatz 10 eine zu seiner Längsachse 13 koaxiale Längsbohrung 14 und eine zu seiner nach dem Einsetzen in den Marknagel senkrecht mit dessen Kannulierung korrespondierende Querbohrung 24 aufweisen.

In Fig. 6 ist eine Variante eines einteiligen Einsatzes 10 dargestellt der aus einem Stift 17 mit konischem erweitertem Kopf 18 besteht.

In Fig. 7 ist eine weitere Variante eines einteiligen Einsatzes 10 dargestellt der aus einem zentral durchbohrten Stift 19 mit mehreren peripheren angeordneten, konischen Erweiterungen 20 besteht, welche in entsprechende Kavitäten 21, respektive in der Längsbohrung 23 im Bereich der Querbohrung 3 im Form eines Klickverschlusses eingreifen können.

## Patentansprüche

1. Chirurgischer Nagel (1), insbesondere intramedullärer Marknagel, mit einer Zentralachse (2), der aus einem Material M mit der Zugfestigkeit F_{z}, der Druckfestigkeit F_{d}, der Dichte ρ₂ und dem Elastizitätsmodul E besteht und mindestens eine quer zur Zentralachse (2) verlaufenden Querbohrung (3) mit dem Querschnittsprofil F und einer Querachse (4) aufweist, wobei das Querschnittsprofil F in Richtung der Zentralachse eine maximale Länge a und senkrecht dazu eine maximale Breite b aufweist und der Marknagel im Bereich der Querbohrung (3) einen Durchmesser D aufweist und der Nagel (1) einen in die Querbohrung (3) einführbaren Einsatz (10) mit der Längsachse (13) umfasst, welcher aus einem Material m besteht, der ein geringeres Elastizitätsmodul e < E aufweist als das Material M, wobei der Einsatz (10) eine in Richtung seiner Längsachse (13) gemessene Länge L aufweist, welche der Bedingung L > 0,2 D gehorcht;
**dadurch gekennzeichnet, dass**
der Einsatz (10) aus einem zentral durchbohrten Stift (19) mit mehreren peripher angeordneten, konischen Erweiterungen (20) besteht, welche in entsprechende Kavitäten (21), respektive in der Längsbohrung (23) im Bereich der Querbohrung (3) in Form eines Klickverschlusses eingreifen können.

2. Nagel (1) nach Anspruch 1, **dadurch gekennzeichnet, dass** L > 0,5 D und vorzugsweise L = D ist.

3. Nagel (1) nach Anspruch 1 oder 2, **dadurch gekennzeichnet, dass** der Einsatz (10) im wesentlichen kongruent zur Querbohrung (3) ist.

4. Nagel (1) nach Anspruch 1, **dadurch gekennzeichnet, dass** der Einsatz (10) eine zu seiner Längsachse (13) koaxiale Bohrung (14) aufweist.

5. Nagel (1) nach einem der Ansprüche 1 bis 3, **dadurch gekennzeichnet, dass** das Elastizitätsmodul e < 0,8 E, vorzugsweise e < 0,7 E ist.

6. Nagel (1) nach einem der Ansprüche 1 bis 5, **dadurch gekennzeichnet, dass** das Material m eine Zugfestigkeit f_{z} < F_{z} aufweist.

7. Nagel (1) nach einem der Ansprüche 1 bis 6, **dadurch gekennzeichnet, dass** das Material m eine Druckfestigkeit f_{d} < F_{d} aufweist.

8. Nagel (1) nach einem der Ansprüche 1 bis 7, **dadurch gekennzeichnet, dass** das Material m ein biokompatibler Kunststoff, vorzugsweise ein Polyethylen oder ein hochmolekulares Polyethylen (HMWPE) ist.

9. Nagel (1) nach einem der Ansprüche 1 bis 8, **dadurch gekennzeichnet, dass** der Kunststoff ein bioresorbierbares Polymer oder Copolymer, vorzugsweise ein Polylactid ist.

10. Nagel (1) nach einem der Ansprüche 1 bis 9, **dadurch gekennzeichnet, dass** die Querbohrung (3) eine Kreisbohrung ist, wobei das Querschnittsprofile F die maximalen Längen a = b aufweist.

11. Nagel (1) nach einem der Ansprüche 1 bis 10, **dadurch gekennzeichnet, dass** die Querbohrung (3) als Langloch ausgebildet ist, wobei das Querschnittsprofile F die maximalen Längen a > b aufweist.

12. Nagel (1) nach einem der Ansprüche 1 bis 11, **dadurch gekennzeichnet, dass** das Material m auch eine geringere Dichte ρ1 als das Material M mit der Dichte ρ2 aufweist, wobei vorzugsweise ρ1 < 0,8 ρ2 ist.

13. Nagel (1) nach einem der Ansprüche 1 bis 12, **dadurch gekennzeichnet, dass** er eine in die Querbohrung (3) durch den Einsatz (10) hindurchführbare Verriegelungsschraube oder einen Verriegelungsbolzen umfasst, dessen Schaft einen Durchmesser d aufweist, welcher den Bedingungen a > d < b gehorcht.

14. Nagel (1) nach einem der Ansprüche 1 bis 13, **dadurch gekennzeichnet, dass** sich die Querbohrung (3) gegen die Oberfläche des Nagels (1) hin erweitert, vorzugsweise in Form eines konischen Abschnittes

15. Nagel (1) nach einem der Ansprüche 1 bis 14, **dadurch gekennzeichnet, dass** er eine Verriegelungsschraube (30) mit einem Schraubenschaft (31) und einem Aussengewinde (32) umfasst, wobei für den Durchmesser d des Schraubengewindes (32) a > d < b gilt, und d vorzugsweise mindestens 5 % kleiner ist als die kleinere der beiden Dimensionen a, b.

16. Nagel (1) nach einem der Ansprüche 1 bis 15, **dadurch gekennzeichnet, dass** der Einsatz (10) aus einem Stift (17) mit konischem erweitertem Kopf (18) besteht.

## Claims

1. A surgical pin (1), in particular an intramedullar pin, with a central axis (2), that is made from a material *M* having a tensile strength F_{z}, compression strength F_{d}, density p₂ and modulus of elasticity E and has at least one transverse bore (3) extending transversely to the central axis (2) and having a cross-section *F* and a transverse axis (4), while the transverse cross-section *F* has a maximum length *a* in the direction of the central axis and at right angle to it a maximum width *b* and the intramedullar pin has a diameter *D* in the region of the transverse bore (3) and the intramedullar pin comprises an insert (10) with the longitudinal axis (13), that can be introduced into the transverse bore (3) and is made from material *m,* that has a smaller modulus of elasticity *e* < E than material *M,* while the insert (10) has a length *L* in the direction of the longitudinal axis (13), that satisfies the condition *L* > 0,2 *D,*
**characterized in that**
the insert (10) consists of a pin (19) with a central through bore and a plurality of tapered expansions (20) provided on the circumference, that can engage corresponding cavities (21), respectively the longitudinal bore (23) in the region of the transverse bore (3) in the form of a click lock.

2. A pin (1) according to claim 1, **characterised in that** L > 0,5 *D* and preferably *L*=*D.*

3. A pin (1) according to claim 1 or 2, **characterised in that** the insert (10) is basically congruent with the transverse bore (3).

4. A pin (1) according to claim 1, **characterised in that** the insert (10) has a bore (14) that is coaxial with its longitudinal axis (13).

5. A pin (1) according to any one of claims 1 to 3, **characterised in that** the modulus of elasticity is *e* < 0,8 E, preferably *e* < 0,7 E.

6. A pin (1) according to any one of claims 1 to 5, **characterised in that** the material *m* has a tensile strength f_{z} < F_{z}.

7. A pin (1) according to any one of claims 1 to 6, **characterised in that** the material *m* has a compression strength f_{d} < F_{d}.

8. A pin (1) according to any one of claims 1 to 7, **characterised in that** the material *m* is a biocompatible synthetic material, preferably a polyethylene or a high-molecular polyethylene (HMWPE).

9. A pin (1) according to any one of claims 1 to 8, **characterised in that** the synthetic material is a bioabsorbable polymer or copolymer, preferably a polylactide.

10. A pin (1) according to any one of claims 1 to 9, **characterised in that** the transverse bore (3) is a circular bore, while the cross-section *F* has the maximum lengths *a* = *b.*

11. A pin (1) according to any one of claims 1 to 10, **characterised in that** the transverse bore (3) is constructed as a slotted hole, while the cross-section *F* has the maximum lengths *a* > *b.*

12. A pin (1) according to any one of claims 1 to 11, **characterised in that** the density p₁ of material *m* is lower than the density p₂ of material *M,* preferably p₁ < 0,8 p₂.

13. A pin (1) according to any one of claims 1 to 12, **characterised in that** it comprises a locking screw or a locking bolt that can be passed through the insert (10) in the transverse bore (3), the shaft of which has a diameter *d,* that satisfies the conditions *a* > *d* < *b.*

14. A pin (1) according to any one of claims 1 to 13, **characterised in that** the transverse bore (3) expands towards the surface of the pin (1), preferably in the form of a tapered section.

15. A pin (1) according to any one of claims 1 to 14, **characterised in that** it comprises a locking screw (20) with a screw shaft (21) and an external thread (22), while *a* > *d* < *b* is valid for the diameter *d* of the screw thread (22), and *d* is preferably be at least 5% smaller than the smaller of the two dimensions *a, b.*

16. A pin (1) according to any one of claims 1 to 15, **characterised in that** the insert (10) is a pin (17) with a tapered expanded head (18).

## Revendications

1. Clou chirurgical (1), en particulier clou intramédullaire comprenant un axe central (2), qui est composé d'un matériau M ayant une résistance à la traction F_{z}, une résistance à la compression F_{d}, une densité ρ2 et un module d'élasticité E, et présente au moins un alésage transversal (3) s'étendant transversalement à l'axe central (2) ayant un profil de section transversale F et un axe transversal (4), dans lequel le profil de section transversale F, dans le sens de l'axe central, présente une longueur maximale a et, perpendiculairement à celui-ci, une largeur maximale b, et le clou intramédullaire présente, dans la zone de l'alésage transversal (3), un diamètre D et le clou (1) comprend un insert (10), ayant un axe longitudinal (13), qui peut être introduit dans l'alésage transversal (3), lequel insert est composé d'un matériau m présentant un module d'élasticité e < E plus faible que celui du matériau M, l'insert (10) présentant une longueur L mesurée dans le sens de son axe longitudinal (13), laquelle remplit la condition L > 0,2 D ;
**caractérisé en ce que**
l'insert (10) est formé par une tige transpercée au centre (19) présentant plusieurs élargissements coniques périphériques (20) qui peuvent s'engager à la manière d'un verrouillage par encliquetage dans des cavités correspondantes (21), respectivement dans l'alésage longitudinal (23), dans la zone de l'alésage transversal (3).

2. Clou (1) selon la revendication 1, **caractérisé en ce que** L > 0,5 D et de préférence L = D.

3. Clou (1) selon la revendication 1 ou 2, **caractérisé en ce que** l'insert (10) est essentiellement congruent à l'alésage transversal (3).

4. Clou (1) selon la revendication 1, **caractérisé en ce que** l'insert (10) présente un alésage (14) coaxial à son axe longitudinal (13).

5. Clou (1) selon l'une des revendications 1 à 3, **caractérisé en ce que** le module d'élasticité est inférieur à 0,8 E(e < 0,8 E), de préférence e < 0,7 E.

6. Clou (1) selon l'une des revendications 1 à 5, **caractérisé en ce que** le matériau m présente une résistance à la traction f_{z} < F_{z}.

7. Clou (1) selon l'une des revendications 1 à 6, **caractérisé en ce que** le matériau m présente une résistance à la compression f_{d} < F_{d}.

8. Clou (1) selon l'une des revendications 1 à 7, **caractérisé en ce que** le matériau m est une matière plastique biocompatible, de préférence un polyéthylène ou un polyéthylène de masse moléculaire élevée (HMWPE).

9. Clou (1) selon l'une des revendications 1 à 8, **caractérisé en ce que** la matière plastique est un polymère ou copolymère biorésorbable, de préférence un polylactide.

10. Clou (1) selon l'une des revendications 1 à 9, **caractérisé en ce que** l'alésage transversal (3) est un alésage circulaire, le profil de section transversale F présentant les longueurs maximales a = b.

11. Clou (1) selon l'une des revendications 1 à 10, **caractérisé en ce que** l'alésage transversal (3) est réalisé en tant qu'alésage oblong, le profil de section transversale F présentant les longueurs maximales a > b.

12. Clou (1) selon l'une des revendications 1 à 11, **caractérisé en ce que** le matériau m présente également une densité ρ₁ inférieure à celle du matériau M ayant la densité ρ₂, dans lequel, de préférence, ρ₁ < 0,8 ρ₂.

13. Clou (1) selon l'une des revendications 1 à 12, **caractérisé en ce qu'**il comprend une vis de verrouillage ou un boulon de verrouillage, pouvant être inséré dans l'alésage transversal (3) à travers l'insert (10), vis ou boulon dont la tige présente un diamètre d qui remplit les conditions a > d < b.

14. Clou (1) selon l'une des revendications 1 à 13, **caractérisé en ce que** l'alésage transversal (3) s'élargit vers la surface du clou (1), de préférence sous la forme d'un segment conique.

15. Clou (1) selon l'une des revendications 1 à 14, **caractérisé en ce qu'**il comprend une vis de verrouillage (30) ayant une tige de vis (31) et un filetage (32), la relation a > d < b s'appliquant au diamètre d du filetage (32), et d étant de préférence inférieur d'au moins 5% par rapport à la plus petite des deux dimensions a, b.

16. Clou (1) selon l'une des revendications 1 à 15, **caractérisé en ce que** l'insert (10) se compose d'une tige (17) présentant une tête élargie conique (18).
